# EUROPÄISCHE PATENTSCHRIFT

(11) **EP 1 370 664 B1**
(45) Veröffentlichungstag und Bekanntmachung des Hinweises auf die Patenterteilung: **02.12.2009**
(21) Anmeldenummer: 02732338.5
(22) Anmeldetag: 18.03.2002
(51) Int. Cl.: C12N 15/68

(54) **GEGEN ABBAU STABILISIERTE NUKLEINSÄURE**
NUCLEIC ACID WHICH IS STABILISED AGAINST DECOMPOSITION
ACIDE NUCLEIQUE STABILISE CONTRE LA DECOMPOSITION

(30) Priorität: 16.03.2001 DE 10113265; 16.03.2001 DE 10145014; 05.10.2001 DE 10151071
(43) Veröffentlichungstag der Anmeldung: 17.12.2003
(73) Patentinhaber: QIAGEN GmbH, 40724 Hilden (DE)
(72) Erfinder: MERK, Helmut, 14195 Berlin (DE); STIEGE, Wolfgang, 14195 Berlin (DE)
(74) Vertreter: Jungblut, Bernhard Jakob
(86) Internationale Anmeldenummer: PCT/DE2002/001048
(87) Internationale Veröffentlichungsnummer: WO 2002/074952

(56) Entgegenhaltungen:
- EP-A- 0 743 367
- EP-A- 1 251 179
- WO-A-95/20676
- IHLE O ET AL: "Efficient purification of DNA fragments using a protein binding membrane." NUCLEIC ACIDS RESEARCH. ENGLAND 15 AUG 2000, Bd. 28, Nr. 16, 15. August 2000 (2000-08-15), Seite E76 XP002230673 ISSN: 1362-4962
- CHIU NORMAN H.L.; CHRISTOPOULOS THEODORE K.: 'Two-site expression immunoassay using a firefly luciferase-coding DNA label' CLINICAL CHEMISTRY Bd. 45, Nr. 10, November 1999, Seiten 1954 - 1959

## Beschreibung

### Gebiet der Erfindung

Die Erfindung betrifft eine Verwendung einer stabilisierte Nukleinsäure. Nukleinsäuren können DNA oder RNA, aber auch PNA sein, einzelsträngig oder doppelsträngig.

### Hintergrund der Erfindung.

Proteine werden für biotechnologische und medizinische Anwendungen in hoher Reinheit, insbesondere aber auch in hoher Menge, i.e. im mg- und g- Maßstab benötigt. Im Falle von größeren Proteinen ist eine klassische Synthese kaum möglich und jedenfalls unwirtschaftlich.

Eine Möglichkeit der Herstellung von Proteinen in größerem Maßstab ist die gentechnische Herstellung. Hierzu wird klonierte DNA, welche für das gewünschte Protein codiert, als Fremd DNA in Form von Vektoren bzw. Plasmiden in Zellen, insbesondere prokaryontische Zellen eingeschleust. Diese Zellen werden dann kultiviert, wobei die durch die Fremd DNA codierten Proteine exprimiert und gewonnen werden. Zwar lassen sich auf diesem Wege bereits beachtliche Mengen an Protein gewinnen, die insofern bekannten Maßnahmen, insbesondere die Klonierung, sind jedoch aufwendig. Zudem sind die Zellen meist nur transient transfiziert und zudem nur in Ausnahmefällen stabil immortalisiert. Eine kontinuierliche Produktion von Protein erfordert daher einen stetigen Nachschub an frischen Zellen, welche wiederum mittels der vorstehend beschriebenen aufwendigen Maßnahmen hergestellt werden müssen.

Ein weiterer Ansatz ist die sogenannte zellfreie in vitro Proteinbiosynthese. Hierbei werden biologisch aktive Zellextrakte eingesetzt, welche von natürlicherweise vorliegenden zellulären Nukleinsäuren weitgehend befreit sind und welche mit Aminosäuren, energieliefernden Substanzen und zumindest einer Nukleinsäure versetzt werden. Die zugesetzte Nukleinsäure codiert dabei für das herzustellende Protein. Wenn als Nukleinsäure DNA eingesetzt wird, ist die Anwesenheit einer DNA-abhängigen RNA-Polymerase erforderlich. Es kann natürlich auch direkt RNA, mRNA, eingesetzt werden. Auf diesem Wege lassen sich nicht nur solche Proteine in kurzer Zeit und mit vergleichsweise moderatem Aufwand herstellen, die auch gentechnisch herstellbar sind, vielmehr können sogar Proteine hergestellt werden, die beispielsweise zelltoxisch sind und folglich mit den üblichen gentechnischen Zellsystemen überhaupt nicht nennenswert exprimierbar sind. Allerdings ist es notwendig, die zugesetzte Nukleinsäure selbst herzustellen, was mittels gentechnischer Methoden wiederum aufwendig ist. Hinzu kommt, daß es oft wünschenwert ist, nicht natürlicherweise mit einer Proteinsequenz verknüpfte regulatorische Sequenzen sowie sonstige Sequenzen, wie Spacer, einzuführen, um die Effizienz der Proteinsynthese zu verbessern.

Eine Alternative zur gentechnischen Herstellung von vollständigen, in der zellfreien Proteinbiosynthese einsetzbaren Nukleinsäuren ist die sogenannte Expressions PCR. In diesen Zusammenhängen spielt die effiziente Einführung regulatorischer Sequenzen (sowie anderer, die Translationseffizienz fördernder Sequenzen) in eine herzustellende Nukleinsäure im Rahmen der Amplifikation eine besondere Rolle. Für die Einführung solcher weiterer Sequenzen in eine Ziel-Nukleinsäure sind sehr lange PCR Primer notwendig. Lange Primer sind einerseits in der Herstellung wiederum aufwendig und erhöhen andererseits die Wahrscheinlichkeit der Generierung inhomogener PCR-Produkte.

Unabhängig von der Herstellungsweise der Nukleinsäuren für die zellfreie Proteinbiosynthese stellt sich die folgende grundsätzliche Problematik. Im Rahmen dieser Synthesemethode für Proteine werden sogenannte Zelllysate verwendet, i.e. Extrakte aus Zellen, welche die für die Proteinsynthese wesentlichen Komponenten und Zellbausteine enthalten. Der Einsatz socher Zelllysate bedingt aber auch, daß in den Ursprungszellen natürlicherweise vorhandene (Exo-) Nukleasen in das Lysat gleichsam verschleppt werden. Diese Nukleasen bewirken einen Abbau der für die Proteinsynthese hergestellten und eingesetzten Nukleinsäuren und reduzieren folglich deren Halbwertszeit und letztendlich somit die Proteinausbeute. Dies stört aus offensichtlichen Gründen. Die gleiche Problematik stell sich natürlich auch im Faller der zellulären Systeme.

### Stand der Technik.

Aus der Praxis ist es bekannt, im Rahmen der zellfreien Proteinbiosynthese Zelllysate zu verwenden, die natürlicherweise eine geringe Menge an (Exo-) Nukleasen enthalten. Ein Beispiel hierfür ist das Escherichia coli S-30 Lysat. Zwar kann dadurch eine gegenüber anderen Lysaten verbesserte Halbwertszeit der intakten Nukleinsäure in dem Synthesesystem und folglich eine Erhöhung der Proteinausbeute bei Einsatz gleicher Menge an Nukleinsäure erreicht werden, jedoch findet dennoch ein Abbau durch Nuklease in störendem Ausmaß statt.

Ebenfalls aus der Praxis ist es bekannt, zum Zwecke der Reinigung ein Ende einer Nukleinsäure mit einem Affinitätsmolekül, beispielsweise Biotin, auszustatten. Biotin ist dann in der Lage an immobilisiertes Streptavidin zu binden, wodurch die Nukleinsäure immobilisiert, von der Lösung und deren sonstigen Komponenten getrennt und danach - gereinigt - wieder von der festen Phase abgelöst wird.

Die Literaturstelle EP 0743367-A betrifft ein System zur Genexpressionsanalyse, wobei Biotin oder Digoxigenin als Marker verwendet werden. Einen ähnlichen Gegenstand beschreibt die Literaturstelle WO 95/20676A. Die Literaturstelle IHLE et al., Nucleic acids research, vol. 28, no. 16, 2000, page E76 beschreibt ein Reinigungsverfahren für DNA-Fragmente wobei Biotin als Label benutzt wird. Die Literaturstelle WO 99/14346A betrifft ein Verfahren zur Stabilisierung von RNA zur intrazellulären Proteinexpression. Die Literaturstelle Joshizawa et al., nucleic acids research, vol. 22, no. 12, page 2217, 1994, beschreibt ein Nuklease-resistentes DNA-Freundfragment und dessen Verwendung in der Proteinsynthese.

### Technisches Problem der Erfindung.

Der Erfindung liegt das technische Problem zugrunde, Nukleinsäuren anzugeben, die beispielsweise bei Einsatz in einem zellfreien Proteinbiosynthesesystem eine verbesserte Halbwertzeit und folglich eine verbesserte Proteinausbeute erreichen.

### Grundzüge der Erfindung.

Zur Lösung dieses technischen Problems lehrt die Erfindung den Gegenstand des Anspruchs 1.

Als volumige Moleküle B sind solche mit einem Molekulargewicht von mehr als 1000, vorzugsweise mehr als 10000, bezeichnet. Zweckmäßigerweise handelt es sich bei den voluminösen Molekülen B um Proteine. Bei den Molekülen A handelt es sich um vergleichsweise kleine Moleküle, welche zumindest eine freie Bindungsstelle für ein zum Moleküle A gepaartes Molekül B aufweisen. Ein Molekül B kann eine, aber auch mehrere Bindungsstellen für ein zum Molekül B gepaartes Molekül A aufweisen. Die Bindung zwischen einem Molekül A und einem Molekül B kann sowohl nicht-kovalent als auch kovalent sein. Die Bindung des Moleküls A an ein endständiges Nukleotid der linearen Sequenz ist zweckmäßigerweise kovalent. Wenn an sowohl am 3' als auch am 5' Ende der linearen Sequenz jeweils ein Molekül A angebracht ist, erfolgt eine Stabilisierung der erfindungsgemäßen Nukleinsäure gegenüber sowohl 3'- als auch 5'-Exonukleasen. An beiden Enden lassen sich Moleküle A anfügen, indem an sowohl den sense als auch den antisense Strang einer doppelsträngigen Nukleinsäure jeweils am 5'-Ende ein Primer mit dem Molekül A anhybridisiert wird.

Die Erfindung beruht auf der Erkenntnis, daß sich mit modifizierten Primern, nämlich solchen, die ein Molekül A trage, auf einfachem Wege mittels PCR und in hoher Menge Nukleinsäuren herstellen lassen, die an zumindest einem Ende ein Molekül A tragen. Eine voluminöse Schutzgruppe zur Verhinderung eines Angriffes von Exonukleasen läßt sich dann in Form des Moleküls B einfach anhängen. Moleküle B können unschwer in der benötigten hohen Menge eingesetzt werden, da hierfür beispielsweise handelsübliche und preiswerte Proteine eingesetzt werden können.

In dem Fall, daß ein Molekül B mehrere Bindungsstellen n für ein zum Molekül B gepaartes Molekül A aufweist, kann es zweckmäßig sein, einen Teil der Bindungstellen, insbesondere n-1 Bindungstellen so abzusättigen, so daß nur noch wenige Moleküle A, insbesondere nur ein Molekül A an einem Molekül B binden. Zum Absättigen der Bindungsstellen des Moleküles B können sowohl nicht an Primer gebundene Moleküle A, als auch andere Moleküle verwendet werden, die an die Bindungsstelle für die Moleküle A des Moleküles B binden. Es versteht sich, daß in der Lösung Moleküle B in der benötigten hohen Menge eingesetzt werden, daß trotz der Absättigung jedes Molekül A eine Molekül B binden kann. Durch die Absättigung der Bindungsstellen des Moleküles B, die für die Bindungen des Moleküles B notwendig sind, kann verhindert werden, daß ein Molekül B mehrere Moleküle A bindet, und es somit zu ein Aggregation der an Moleküle A gebundenen Primer an einem Molekül B kommt, was die Amplifikation der Nukleinsäurebasissequenzen stören kann. Auch kann es zu einer Zusammenlagerung mehrerer amplifizierten Nukleinsäurebasissequenzen über die durch die Primer angefügten Moleküle A an einem Molekül B kommen, was die Transkription und/oder Translation der Nukleinsäurebasissequenz erschweren und somit die translatierte Menge des durch die Nukleinsäurebasissequenz codierten Proteins verringern kann.

Um einer Zusammenlagerung mehrerer amplifizierten Nukleinsäurebasissequenzen über die durch die Primer angefügten Moleküle A an einem Molekül B zu vermindern, insbesondere zu verhindern, kann es von Vorteil sein, die Transkription/Translation in zeitlich direktem Anschluß an die Amplifikation durchzuführen.

Mit der Erfindung wird erreicht, daß Exonukleasen nicht mehr oder in nur sehr reduziertem Maße die zur Proteinsynthese hergestellten und eingesetzten Nukleinsäuren angreifen und abbauen können. Dadurch wird die Halbwertszeit der in der Herstellung aufwendigen und folglich teuren Nukleinsäuren in einem Expressionssystem beachtlich erhöht mit der Folge einer ebenso beachtlich erhöhten Ausbeute an Protein bei mengenmäßig gleichem oder sogar reduzierten Einsatz der Nukleinsäuren.

Ein Verfahren zur Herstellung einer erfindungsgemäß verwendeten Nukleinsäure, hat die folgenden Verfahrensstufen: 1) es wird eine lineare Sequenz enthaltend die Komponenten a) und b) hergestellt, 2) die lineare Sequenz aus Stufe 1) wird mittels PCR amplifiziert, wobei zumindest ein Primerpaar eingesetzt wird, welches das Molekül A trägt, 3) das Produkt aus Stufe 2) wird mit einer Lösung enthaltend Moleküle B inkubiert.

Eine besonders vorteilhafte Ausführungsform eines solchen Verfahrens ist ein Verfahren zur präparativen Herstellung von langen Nukleinsäuren mittels PCR, mit den folgenden Hybridisierungsschritten: a) eine Nukleinsäurenbasissequenz wird am 3' und 5' Ende mit jeweils einem Adapterprimer hybridisiert, b) das Produkt aus Stufe a) wird am 3' und 5' Ende mit jeweils einem Verlängerungsprimer, welcher eine Verlängerungssequenz enthält, hybridisiert, wobei aus der Nukleinsäurebasissequenz eine an dem 3' und dem 5' Ende der Nukleinsäurebasissequenz um die Verlängerungssequenzen verlängerte und amplifizierte Nukleinsäuresequenz gebildet wird und wobei vorzugsweise die in einer letzten Amplifikationsstufe eingesetzten Primer ein Molekül A tragen.

Als Nukleinsäurebasissequenz ist eine Sequenz bezeichnet, die für ein Protein codiert. Dabei kann es sich insbesondere um ein Gen handeln, aber auch um Sequenzen aus intronlosen Genomen. Bei den Verlängerungssequenzen kann es sich insbesondere um Sequenzen handeln, die eine regulatorische Sequenz umfassen und/oder um Sequenzen, welche eine ribosomale Bindungssequenz enthalten. Die Adapterprimer sind vergleichsweise kurz. Eine Teil einer Adaptersequenz ist spezifisch für die Nukleinsäurebasissequenz, ein weiterer Teil ist konstant und hybridisiert jeweils einer Verlängerungssequenz.

Hieraus folgt, daß für unterschiedliche Nukleinsäurebasissequenzen nicht jeweils "passende" lange Verlängerungssequenzen eingesetzt werden müssen. Vielmehr müssen lediglich die vergleichsweise kurzen Adapterprimer auf eine definierte Nukleinbasissequenz abgestimmt werden, während die Verlängerungssequenzen gleichsam universell sein können, i.e. für verschiedene Nukleinsäurebasissequenzen sind stets die gleichen bzw. einige wenige ausgewählte Verlängerungsequenzen einsetzbar. Somit können die relativ aufwendig herzustellenden Verlängerungsequenzen einer breiten Nutzung zugeführt werden und für eine spezifische Nukleinsäurebasissequenz müssen lediglich die Adaptersequenzen hergestellt werden. Dies ist jedoch wenig aufwendig, da die Adaptersequenzen vergleichsweise kurz sein können.

Dies erlaubt es beispielsweise, sowohl eine regulatorischen Sequenz als auch eine ribosomalen Bindungssequenz, jeweils über einen der Verlängerungsprimer, an eine Nukleinsäurebasissequenz anzufügen, und zwar sogar in einem PCR Schritt. So kann eine Nukleinsäure erhalten werden, die eine besonders hohe Transskriptions- und/oder Translationseffizienz in einem prokaryontischen System der zellfreien Proteinbiosynthese ergibt.

Ein besonderer Vorteil dieser Verfahrensweise ist, daß es sich um ein allgemein anwendbares Verfahren für beliebige codierende Sequenzen handelt.

Bezüglich der Verfahrensstufen der zellfreien Proteinbiosynthese wird auf die Ausführungsbeispiele verwiesen, aus welchen der Fachmann ohne weiteres die grundlegenden Merkmale verallgemeinern kann. Ausführungsformen der Erfindung.

Hinsichtlich der erfindungsgemäß verwendeten Nukleinsäure ist es nötig, dass beide Enden der linearen Sequenz mit je einem Molekül A verbunden sind, da dann vollständige Stabilisierung beider Enden der Sequenz gegenüber Exonukleasen gewährleistet ist.

Um die Expression nicht durch die voluminösen Moleküle B zu behindern kann es sich empfehlen, wenn zwischen den Komponenten a und/oder b und dem Molekül A oder den Molekülen A eine Spacersequenz eingerichtet ist.

Im Einzelnen kann jedes Molekül A mit je einem Molekül B verbunden sein oder es können beide Moleküle A mit einem einzigen, zumindest zwei Bindungstellen für ein Molekül A aufweisenden Molekül B verbunden sein. In ersterem Falle wird ein lineares Produkt erhalten. In letzterem Fall wird ein cirkularisiertes Produkt erhalten, welches hinsichtlich der Stabilität noch weiter verbessert sein kann.

Das Molekül A kann Biotin oder Digoxygenin und das Molekül B kann Avidin, Streptavidin oder Anti-Digoxygenin-Anti-körper sein. Dies sind in großen Mengen und preiswert erhältliche Handelsprodukte.

In dem Fall, daß das Molekül B Avidin oder Streptavidin ist, kann es zwegmäßig seien, einen Teil der Bindungstellen n, insbesondere n-1 Bindungstellen so abzusättigen, so daß nur noch wenige Moleküle Biotin, insbesonder nur ein Molekül Biotin an einem Molekül Avidin oder Streptavidin binden..Zum Absättigen der Bindungstellen des Avidins oder Streptavidins können sowohl nicht an einen Primer gebundenes Biotin, als auch andere Moleküle verwendet werden, die an die Bindungsstelle für das Biotin am Avidin oder Streptavidin binden. Es versteht sich, daß in der Lösung die Moleküle Avidin und/oder Streptavidin in der benötigten hohen Menge eingesetzt werden, daß trotz der Absättigung jedes Molekül Biotin eine Molekül Avidin oder Streptavidin binden kann. Durch die Absättigung der Bindungen von Avidin oder Streptavidin für die Bindungen des Biotins kann verhindert werden, daß ein Molekül Avidin oder Streptavidin mehrere Moleküle Biotin bindet, und somit es zu ein Aggregation der an Biotin gebundenen Primer an einem Molekül Avidin oder Streptavidin kommt, da dadurch die Amplifikation, Transkription oder Translation der Nukleinsäurebasissequenzen gestört werden kann.

Im Rahmen des Verfahrens zur Herstellung einer erfindungsgemäß verwendeten Nukleinsäure ist von selbstständiger Bedeutung eine Weiterbildung, wobei das Produkt aus der Stufe b) in einer Stufe c) am 3' Ende und am 5' Ende mit jeweils einem Amplifikationsprimer hybridisiert werden kann, wobei eine amplifizierte Nukleinsäureendsequenz gebildet wird. Es versteht sich, daß dann die Primer der Stufe c) mit dem Molekül A ausgestattet sind. Auch die Amplifikationsprimer sind einerseits vergleichsweise kurz und universell einsetzbar und folglich leicht verfügbar. Mittels der Amplifikationsprimer können zudem weitere (kürzere) Sequenzen an den Enden angefügt werden, welche die Translationseffizienz weiter erhöhen. Auch sind mittels der kurzen Amplifikationsprimer auch andere Variationen und Modifikationen an den Enden der Nukleinsäuren mit wenig Aufwand einführbar. Dies ist insbesondere deshalb von Vorteil, da damit für Variationen und Modifikationen keine unterschiedlichen Verlängerungsprimer hergestellt zu werden brauchen, was wiederum in störendem Maße aufwendig wäre.

Insbesondere kann erfindungsgemäß ein Biotinrest am 5'-Ende des Amplifikationsprimers gekoppelt sein. Hierdurch wird nach Inkubation der Nukleinsäureendsequenz mit Biotin-bindendem Streptavidin eine gegen Exonuklease-Abbau stabilisierte Nukleinsäureendsequenz erhalten, welche eine Erhöhung der Halbwertszeit in einem in vitro Proteinbiosynthesesystem gegenüber einer nicht stabilisierten Nukleinsäureendsequenz um ein Vielfaches, typischerweise mehr als 5-fach, beispielsweise von ca. 15 min. auf ca. 2 h, aufweist. Es werden Stabilitäten von linearen Konstrukten erreicht, welche mit jenen von klassischen zirkulären Plasmiden vergleichbar sind und diese insofern praktisch gleichwertig ersetzen können.

Es mag angemerkt werden, daß ein Molekül A auch eine Doppel- oder Mehrfachfunktion aufweisen, beispielsweise gleichzeitig als Ankergruppe funktionieren kann.

Die Adapterprimer enthalten typischerweise < 70, insbesondere 20 - 60, Nucleotide. Die Verlängerungsprimer enthalten typischerweise ≥ 70, auch 90 und mehr, Nucleotide. Die Amplifikationsprimer wiederum enthalten typischerweise < 70, meist < 30, Nukleotide, typischerweise > 9 Nukleotide. Lediglich die Adapterprimer müssen an eine definierte Nukleinsäurebasissequenz spezifisch angepaßt werden, was im Lichte der relativ kurzen Sequenzen mit wenig Aufwand verbunden ist.

Vorteilhafterweise werden die Schritte a), b) und optional der Schritt c) in einer PCR Lösung enthaltend die Nukleinsäurebasissequenz, die Adapterprimer, die Verlängerungsprimer und optional die Amplifikationsprimer durchgeführt. Es handelt sich dann um eine Einstufen PCR mit insgesamt 6 Primern, zwei Adaptersquenzen, zwei Verlängerungssequenzen und zwei Amplifikationssequenzen. Dabei reicht es, die Adapterprimer und Verlängerungsprimer in geringen Konzentrationen einzusetzen und insofern auch nur eine geringe Menge an Zwischenprodukt zu erzeugen. Das Zwischenprodukt braucht zudem nicht homogen vorliegen, wodurch aufwendige Optimierungen entfallen können. Aufgrund der Kürze der Amplifikationsprimer sind auch bei der Amplifikation auf die hohe Menge an Nukleinsäureendsequenz keine Optimierungen erforderlich.

Alternativ zu der vorstehenden Ausführungsform ist von selbstständiger Bedeutung eine Variante, die mit zwei PCR Stufen arbeitet. Dabei werden die Schritte a) und b) in einer Verfahrenstufe A) in einer Vor-PCR Lösung enthaltend die Nukleinsäurebasissequenz, die Adapterprimer und die Verlängerungsprimer für eine definierte erste Zyklenzahl durchgeführt und wird der Schritt c) in einer Verfahrenstufe B) in einer Haupt-PCR Lösung enthaltend das PCR Produkt aus der Stufe A) und die Amplifikationsprimer für eine definierte zweite Zyklenzahl durchgeführt. Dabei kann die Stufe A) in einem Reaktionsvolumen durchgeführt werden, welches 1/2 bis 1/10 des Reaktionsvolumens der Stufe B) beträgt. In der Stufe A) entsteht dann, bedingt durch das geringere Volumen eine höhere Konzentration an Zwischenprodukt bzw. es kann deutlich weniger Nukleinsäurebasissequenz eingesetzt werden. Mit der Verdünnung mittels PCR-Ansatzvolumen beim Übergang von der Stufe A) zur Stufe B) werden wiederum die Adapterprimer sowie die Verlängerungsprimer stark verdünnt mit der Folge einer Erhöhung der Wahrscheinlichkeit des Einbaus von Variationen und/oder Modifikationen in die Nukleinsäureendsequenzen über die Amplifikationsprimer.

Im Einzelnen kann in der ersten vorstehenden Alternative so verfahren werden, daß die PCR in einem Reaktionsvolumen von 10 bis 100 µl, vorzugsweise 20 bis 40 µl, mit 0,01 bis 100 pg, vorzugsweise 1 bis 50 pg, Nukleinsäurebasissequenz, 0,05 bis 10 µM, vorzugsweise 0,1 bis 5 µM, Adapterprimer und 0,005 bis 0,5 µM, vorzugsweise 0,001 bis 0,1 µM, Verlängerungsprimer durchgeführt wird, wobei nach einer definierten Anfangszyklenzahl 0,01 bis 10 µM, vorzugsweise 0,1 bis 10 µM, Amplifikationsprimer zugegeben werden, und wobei mittels einer definierte Anzahl anschließender Zyklen die amplifizierte Nukleinsäureendsequenz hergestellt wird. In der zweiten vorstehenden Alternative empfehlen sich die folgenden Reaktionsbedingungen: Stufe A): Reaktionsvolumen < 10 µl; 0,001 bis 50 pg, vorzugsweise 0,01 bis 5 pg, Nukleinsäurebasissequenz; 0,05 bis 10 µM, vorzugsweise 0,1 bis 5 µM, Adapterprimer und 0,05 bis 10 µM, vorzugsweise 0,1 bis 5 µM, Verlängerungsprimer; erste Zyklenzahl 10 bis 30, vorzugsweise 15 bis 25, Stufe B) : Reaktionsvolumen 10 bis 100 µl, vorzugsweise 15 bis 50 µl, erhalten durch Ergänzung der Lösung aus Stufe A) mit PCR-Ansatzlösung; 0,01 bis 10 µM, vorzugsweise 0,1 bis 5 µM, Amplifikationsprimer; zweite Zyklenzahl 15 bis 50, vorzugsweise 20 bis 40.

Erfindungsgemäß verwendete Nukleinsäuren sind einsetzbar für die zellfreie in vitro Proteinbiosynthese, insbesondere in prokaryontischen Systemen, vorzugsweise in einem Translationsystem aus Escheria coli D10.

Die erfindungsgemäße Verwendung ist vorteilhafterweise einsetzbar zur selektiven Amplifikation einer definierten Nukleinsäurebasissequenz aus einer Nukleinsäurebibliothek. Dies ermöglicht eine Charakterisierung von Gensequenzen, wobei die Gensequenz als Nukleinsäurenbasissequenz eingesetzt wird und wobei das erhaltene Protein hinsichtlich Struktur und/oder Funktion analysiert wird. Der Hintergund dieses Aspekts ist, daß für viele Gene zwar die Sequenzen bekannt sind, nicht jedoch die Struktur und Funktion des dadurch codierten Proteins. Somit können Elemente einer Genbibliothek, zu welchen lediglich die Sequenz als solche bekannt ist, auf ihre Funktion in einem Organismus untersucht werden. Die Untersuchung der Struktur und Funktion des erhaltenen Proteins folgt dabei üblichen Arbeitsmethoden der Biochemie.

Mittels des erfindungsgemäßen Verfahrens lassen sich Nukleinsäuren gewinnen, welche eine für ein Protein codierenden Nukleinsäurebasissequenz und eine ribosomale Bindungssequenz sowie eine oder mehrere Sequenzen aus der Gruppe bestehend aus "Promotorsequenz, Transskriptionsterminatorsequenz, Expressionsenhancersequenz, Stabilisierungssequenz und Affinitytagsequenz" enthalten. Eine Affinitytagsequenz codiert für eine Struktur, welche eine hohe Affinität für (meist immobilisierte) Bindungsstellen in Trennsystemen zur Aufreinigung aufweist. Dadurch ist eine leichte und hochaffine Abtrennung von Proteinen, welche das Affinitytag nicht enthalten, möglich. Ein Beispiel hierfür ist Strep-tag II, eine Peptidstruktur aus 8 Aminosäureresten mit Affinität zu StrepTactin. Eine Stabilisierungssequenz codiert für eine Struktur, welche entweder selbst, oder nach Bindung mit einem für die Struktur spezifischen Bindungsmolekül, eine Stabilisierung gegen Degradation, insbesondere durch Nukleasen, bewirkt. Eine solche Stabilisierungssequenz kann an einem nicht mit einem Molekül A ausgestatteten Ende angebracht sein. Eine Expressionsenhancersequenz steigert die Translationseffizienz gegenüber einer Nukleinsäure ohne Expressionsenhancersequenz. Dabei kann es sich beispielsweise um (nicht translatierte) Spacer handeln. Eine Transkriptionsterminatorsequenz terminiert die RNA-Synthese. Ein Beispiel hierfür ist der T7 Phage Gen 10 Transskriptionsterminator. Transskriptionsterminatorsequenzen können auch gegen Degradation durch 3' Exonukleasen stabilisieren. Vorteilhafte relative Anordnungen der vorstehenden Sequenzelemente zueinander lassen sich aus den folgenden Ausführungsbeispielen verallgemeinern.

Im Folgenden wird die Erfindung anhand von lediglich bevorzugte Ausführungsformen darstellenden Beispielen näher erläutert.

### Methoden:

### PCR:

Die PCR wurde in einem in den Beispielen quantifizierten Reaktionsvolumen mit 10 mM Tris-HCl (pH 8,85 bei 20°C), 25 mM KCl 5 mM (NH₄)₂SO₄, 2 mM MgSO₄, 0,25 mM jeder dNTP, 3 U Pwo DNA Polymerase (Roche) sowie der in den Beispielen angegebenen Menge Nukleinsäurebasissequenz durchgeführt. Die Zyklen wurden für 0,5 min. bei 94°C, 1 min. bei 55°C und 1 min. bei 72°C durchgeführt.

### In vitro Expression:

In vitro Experimente wurden gemäß der Literaturstelle Zubay, G.; Annu. Rev. Genet. 7:267-287 (1973) mit den folgenden Modifikationen durchgeführt. Das Escherichia coli S-30 Lysat wurde mit 750 U/ml T7 Phagen RNA Polymerase (Stratagene) und 300 µM [¹⁴C]Leu (15 dpm/pmol, Amersham) supplementiert. PCR Produkte und Kontrollplasmide wurden in Konzentrationen von 1 nM bis 15 nM eingesetzt. Die Reaktionen wurden bei 37°C durchgeführt, wobei der Verlauf dadurch verfolgt wurde, daß zu subsequenten Zeitpunkten 5 µl Aliquoten der Reaktionsmischung entnommen und der Einbau von [¹⁴C]Leu mittels TCA Präzipitation abgeschätzt wurde. Weitere 10 µl Aliquoten wurden zwecks Analyse des synthetisierten Proteins mittels SDS-PAGE, gefolgt von einer Autoradiographie in einem Phosphoimager-System (Molecular Dynamics) entnommen.

### Plasmid Konstruktion:

Ein high copy Derivat des Plasmids pET BH-FABP (Specht, B. et al.; J. Biotechnol. 33:259-269 (1994)), welches für bovine heart fatty acid bindung protein codiert wurde konstruiert, pHMFA genannt. Ein Fragment von pET BH-FABP wurde durch Verdau mit den Endonukleasen SphI und EcoRI erzeugt und in den Vektor pUC18 insertiert. Bezüglich der Sequenzen, welche für die Synthese von H-FABP relevant sind, ist das Plasmid pHMFA identisch mit dem Originalplasmid. Es sei angemerkt, daß das linearisierte Plasmid sich nicht besser als das circuläre Plasmid verhält.

### Konstruktion von Nukleinsäuren mit verschiedenen Sequenzbereichen stromaufwärts des Promotors:

Das Plasmid pHMFA diente als Matrize für die Konstruktion von Nukleinsäuren mit verschiedenen Sequenzbereichen upstream des Promotors. Die Konstrukte (siehe Beispiele) FA1, FA2 und FA4 mit 0, 5 und 249 Basenpaaren stromaufwärts des Promotors wurden mit den Primern P1, C1 und P2 sowie mit dem downstream Primer P3 generiert. Das Konstrukt FA3 mit einem Sequenzbereich von 15 Basenpaaren stromaufwärts des Promotors wurde durch Verdau von FA4 mit der Endonuklease Bgl II erhalten. Das Kontrollplasmid pHMFA(EcoRV) mit einem Sequenzbereich von 3040 Basenpaaren wurde durch Verdau des Plasmids mit EcoRV erhalten. Alle Produkte wurden gereinigt durch Agarose Gel Elektrophorese, gefolgt von Gel Extraktion mittels des "High Pure PCR Product Purification Kit".

### Affinitätsreinigung:

Die Reinigung des fatty acid binding protein enthaltend Strep-tag II (Voss, S. et al.; Protein Eng. 10:975-982 (1997)) wurde mittels Affinitätschromatographie gemäß Herstellervorschrift (IBA Göttingen, Deutschland) durchgeführt mit der Abweichung eines reduzierten Volumens der Affinitätssäule (200 µl). Die Reaktionsmischung der gekoppelten Transkription/Translation wurde kurz zentrifugiert und dann auf der Säule aufgetragen. Isolierte Fraktionen wurden durch TCA Präzipitation und Autoradiographie nach SDS-PAGE (s.o.) analysiert.

### H-FABP Aktivitätsassay:

Die vollständige Reaktionsmischung mit in vitro synthetisiertem H-FABP wurde auf die Aktivität der Bindung von Oleinsäure untersucht. Verschiedene Volumina (0 - 30 µl) wurden mit Reaktionsmischung ohne H-FABP auf 30 µl aufgefüllt und mit Translationpuffer (50 mM HEPES pH 7,6, 70 mM KOAc, 30 mM NH₄Cl, 10 mM MgCl₂, 0,1 mM EDTA, 0,002 % NaN₃) auf ein Endvolumen von 120 µl verdünnt. Nach Zugabe von 2 µl 5 mM [9,10(n)-³H] Oleinsäure (Amersham) mit einer spezifischen Aktivität von 1000 dpm/pmol wurden die Proben für eine Stunde bei 37°C inkubiert. 50 µl der Proben wurden zur Entfernung ungebundener Oleinsäure mittels Gelfiltration (Micro Bio Spin Chromatographie Columns; Bio-Rad) eingesetzt. Die ³H Radioaktivität der eluierten Fraktionen wurde mittels eines Scintillationszählers gemessen.

### Analyse der Stabilität der Nukleinsäuren:

Radioaktiv markierte Nukleinsäuren wurden gemäß den vorstehenden Bedingungen synthetisiert, jedoch in Gegenwart von 0,167 µCi/µl [α-³⁵S] dCTP. Die markierten Nukleinsäuren wurden in einer gekoppelten Transskription/Translation, Reaktionsvolumen 400 µl, eingesetzt. 30 µl Aliquoten wurden an aufeinanderfolgenden Zeitpunkten entnommen. Nach der Zugabe von 15 µg Ribonuklease A (DNAse-frei, Roche) wurden diese für 15 min. bei 37°C inkubiert. Eine weitere Inkubation für 30 min. bei 37°C wurde nach Zugabe von 0,5 % SDS, 20 mM EDTA und 500 µg/ml Proteinase K (Gibco BRL) in einem Gesamtreaktionsvolumen von 60 µl durchgeführt. Verbleibende PCR Produkte wurden weiter gereinigt mittels Ethanolfällung und wurden danach einer denaturierenden Elektrophorese (5,3% Polyacrylamid, 7 M Harnstoff, o,1 % SDS, TBE) unterzogen. Das getrocknete Gel wurde zur Quantifizierung der Radioaktivität durch ein Phosphoimager System (Molecular Dynamics) laufen gelassen.

### Sequenzen:

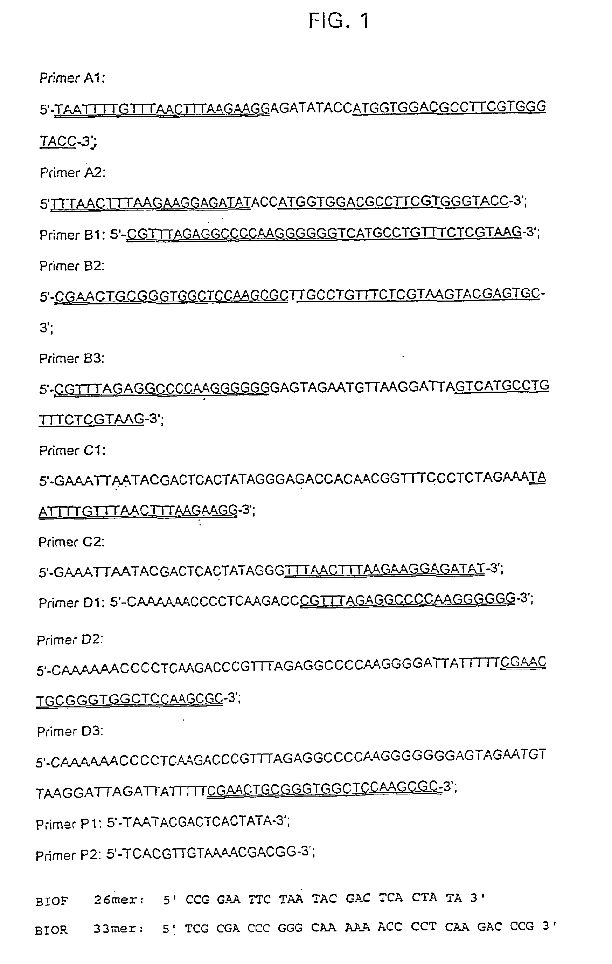

Eingesetzte Primersequenzen sind in der Figur 1 dargestellt.

### Beispiel 1: PCR mit vier Primern

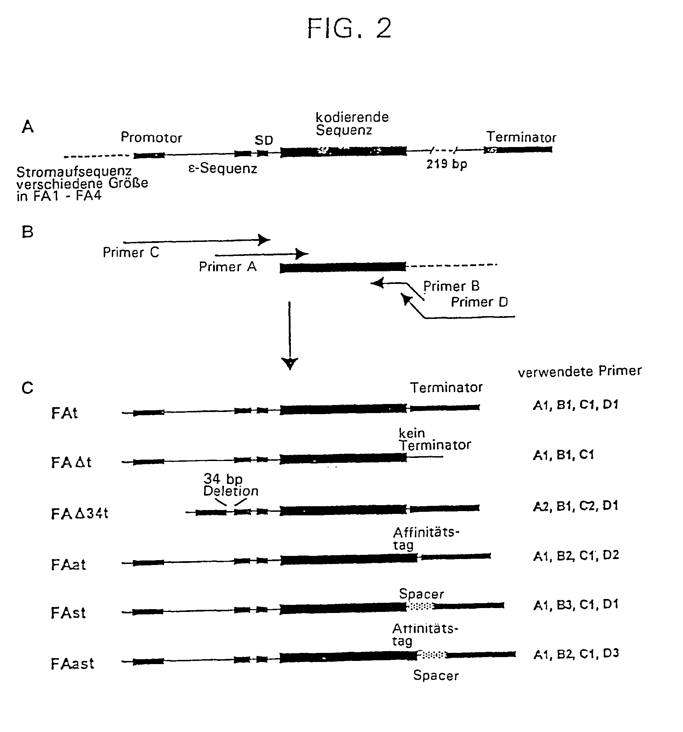

In der Figur 2 ist eine schematische Darstellung einer erfindungsgemäßen Einstufen-PCR mit vier Primern dargestellt. Mittig erkennt man zunächst die für ein Protein codierende Nukleinsäurebasissequenz, welche die vollständige Codierungssequenz für H-FABP (homogeneous and functionally active fatty acid binding protein from bovine heart), erhalten als ein 548 bp Restriktionsfragment aus pHMFA durch Verdau mittels der Endonukleasen Ncol und BamHI, umfaßt (sowie eine 150 bp Sequenz am 3'-Ende, welche weder translatiert wird, noch zu einem Adapterprimer oder Verlängerungsprimer complementär ist). Hieran werden die beiden Adapterprimer A und B hybridisiert, welche mit den Enden der Nukleinsäurebasissequenz homologe Enden umfassen. Der Adapterprimer A enthält weiterhin eine ribosomale Bindungssequenz. An die außenliegenden Enden der Adapterprimer A und B werden die Verlängerungsprimer C und D hybridisiert. Der Verlängerungprimer C umfaßt die T7 Gen 10 Leadersequenz einschließlich des T7 Transskriptionspromotors sowie optional upstream eine Sequenz von beispielsweise 5 Nukleotiden. Der Verlängerungsprimer D umfaßt die T7 Gen 10 Terminatorsequenz.

### Beispiel 2: Effizienz der H-FABP Synthese in Abhängigkeit des Sequenzbereiches upstream des Promotors.

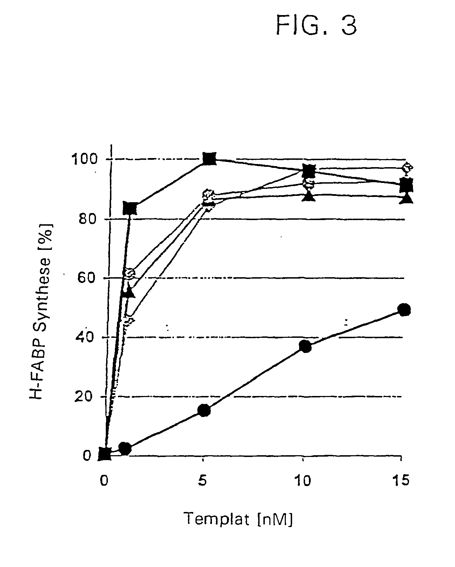

Vier PCR Produkte (FA1 bis FA4) mit verschiedenen Sequenzbereichen upstream des Promotors (0, 5, 15, 250 Basenpaare) und das linearisierte Kontrollplasmid pHMFA(EcoRV) mit 3040 bp upstream des Promotors wurden in verschiedenen Konzentrationen (1, 5, 10 und 15 mM) auf in vitro Transskription/Translation untersucht. Die Figur 3 zeigt, daß alle Sequenzbereiche, außer 0 (untere Kurve), eine Erhöhung der Proteinsynthese bewirken. Bereits 5 Basenpaare reichen aus.

### Beispiel 3: Verbesserung der H-FABP Synthese durch Phage T7 Gen 10 Transskriptionsterminator/5' leader Sequenz Phage T7 Gen 10.

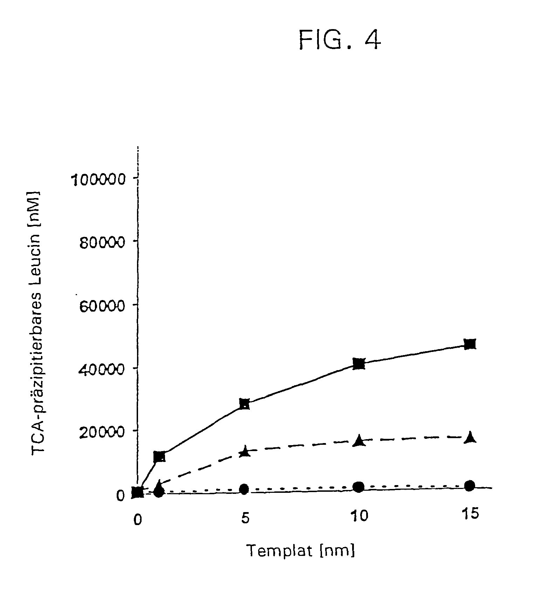

In der Figur 4 erkennt man, daß durch den Phage T7 Gen 10 Transskriptionsterminator die Synthese um zumindest einen Faktor 2,8 verbessert werden kann. Die Dreiecke stehen für FAΔt und die Quadrate für FAt (siehe auch Fig. 2).

Weiterhin erkennt man in der Figur 4, daß eine Deletion von 34 bp zwischen dem Transskriptionsanfang und der epsilon-Sequenz (Olins, P.O. et al.; Escherichia coli. J. Biol. Chem. 264:16973-16976 (1989) zu einer Unterdrückung einer Produktbildung führt. Die Kreise stehen für diese Variante FAΔ34 (siehe auch Fig. 2).

### Beispiel 4: Einfluß der Lage der Transskriptionsterminatorsequenz.

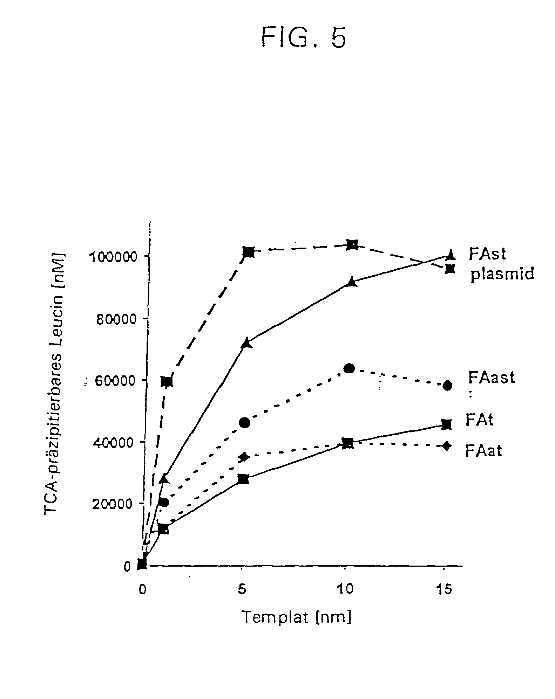

Zur Untersuchung des Einflusses der Lage der Terminatorsequenz wurden die Produkte FAst und FAast (siehe Fig. 2) erzeugt. Beide sind identisch mit FAt und FAat, außer daß eine 22 bp Spacersequenz zwischen dem Stopcodon und dem Terminator mittels unterschiedlicher Primer eingeführt wurde. In der Fig. 5 erkennt man, daß die Spacersequenz eine etwa 2-fache Erhöhung der Expression bewirkt.

In der Figur 5 ist aber auch aus einem Vergleich von FAt und FAat weiterhin zu erkennen, daß ein Affinitytag kaum Einfluß auf die Expression hat.

### Beispiel 5: PCR aus einer komplexen DNA Mischung.

Die Effektivität und Spezifität des erfindungsgemäßen Verfahrens wurde in Gegenwart einer hohen Menge an kompetitiver DNA untersucht. Eine PCR für FAst wurde entsprechend der vorstehenden Beschreibungen ausgeführt mit den folgenden Ausnahmen: die Nukleinsäurebasissequenz wurde in Konzentrationen von 0,16 bis 20 pg/50 µl Reaktorvolumen durchgeführt und die Reaktionen wurden mit 0,83 µg chromosomaler DNA aus Escherichia coli, ultraschallbehandelt für 5 min., supplementiert. Es wurde gefunden, daß weder die Qualität noch die Quantität des PCR Produkts durch die Anwesenheit eines 5-millionenfachen Überschusses an kompetitiver DNA beeinflußt wurde.

### Beispiel 6: Affinitätsreinigung mit Strep-tag II.

Eine Reaktionsmischung mit 10 µg des radioaktiv markierten FAast wurde der Affinitätsreinigung unterworfen. Etwa 81 % des aufgetragenen Materials wurde von der Säule erhalten und 67 % konnten als reines Produkt in den Elutionsfraktionen gewonnen werden (berechnet aus TCA Präzitipation der Fraktionen der Affinitätssäule).

### Beispiel 7: Aktivität des PCR Produkts.

Proben von H-FABP, synthetisiert entweder mittels des Plasmids oder als PCR Produkt FAast, wurden miteinander hinsichtlich der Bindungsaktivität für Oleinsäure untersucht. Nach der Transskription/Translation wurden verschiedene Volumina mit 0 bis 330 pmol nicht-markierten H-FABP in einem Bindungsassay gemäß der vorstehenden Beschreibung zu Methoden untersucht. Die Aktivitäten wurden als identisch gefunden, unabhängig von dem Herstellungsweg.

### Beispiel 8: Stabilität des PCR Produkts.

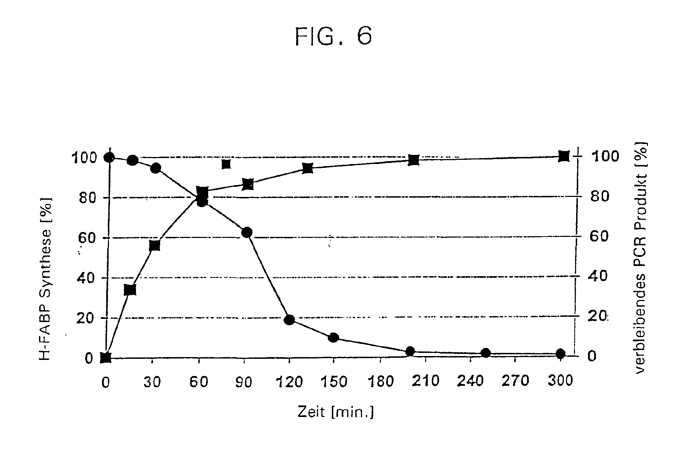

Zur Untersuchung, ob die Stabilität des PCR Produkts möglicherweise die Effektivität der Expression begrenzen könnte, wurde die Abnahme des PCR Produktes FAast gemessen. Es wurde hierfür das radioaktiv markierte Produkt eingesetzt. In bestimmten Zeitabständen wurden Aliquoten des Reaktionsgemisches entnommen und mittels denaturierender Polyacrylamid Gelelektrophorese untersucht. Die Menge am verbleibendem PCR Produkt wurde durch Scannen der Radioaktivität des Gels quantifiziert und mit dem Zeitverlauf der Proteinsynthese, gemessen durch Scannen der Radioaktivität von H-FABP im Gel nach Auftrennung der Reaktionsmischungen mittels SDS-PAGE, verglichen. Die Ergebnisse sind in der Figur 6 dargestellt. Man erkennt, daß die Halbwertzeit des PCR Produkts ca. 100 min. beträgt, was dem Einlauf der H-FABP Synthese in ein Plateau entspricht.

### Beispiel 9: Optimierte Bedingungen für eine PCR mit vier Primern.

In der Tabelle I sind optimierte Bedingungen für eine PCR mit vier Primern in einem Reaktionsvolumen von 25 µl zusammengefaßt.

**Tabelle I**

| a) Reaktionskomponenten | | |
|---|---|---|
| Reaktionskomponente | | Konzentration in der Reaktion |
| PCR-Puffer für Pwo-Polymerase (Roche) | | gemäß Hersteller |
| Desoxynucleotidtriphosphate dATP, dCTP, dGTP und dTTP | | 0,25 mM |
| Adapterprimer a (55 Nucleotide) | | 0,1 µM |
| Adapterprimer b (51 Nucleotide) | | 0,1 µM |
| Verlängerungsprimer c (75 Nucleotide) | | 0,4 µM |
| Verlängerungsprimer d (95 Nucleotide) | | 0,4 µM |
| Templat: codierende Sequenz für fatty acid binding protein Restriktionsfragment aus pHM18FA (NcoI/BamHI) | | 10 pg /25 µl |
| Pwo DNA-Polymerase (Roche) | | 1,5 U / 25µl |

| b) Temperaturprogramm | | |
|---|---|---|
| Temperaturzyklus | | |
| Segment 1 | 30 sek | 94°C |
| Segment 2 | 60 sek | 55°C |
| Segment 3 | 60 sek | 72°C |
| 60 Zyklenwiederholungen | | |

### Beispiel 10: PCR mit sechs Primern.

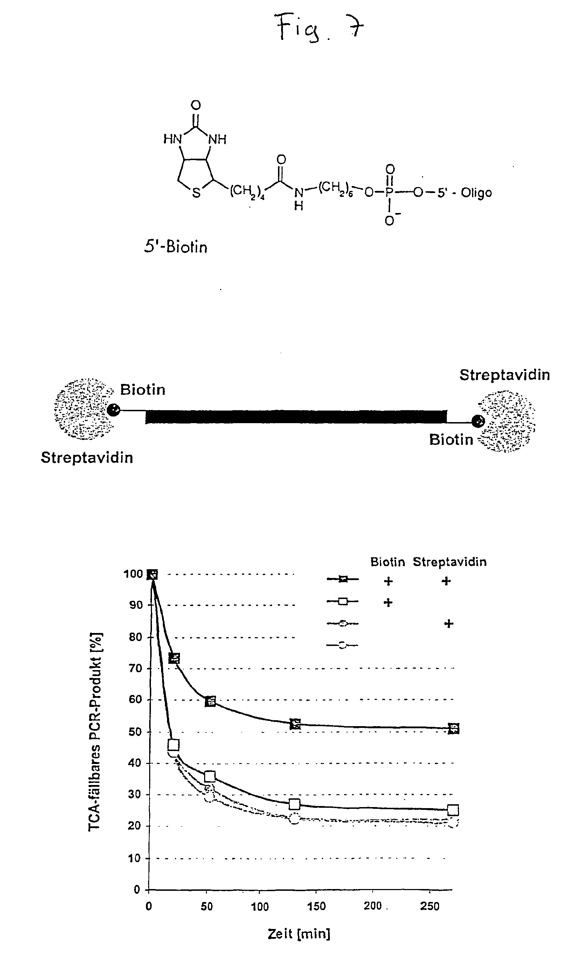

Mit den Materialien aus Beispiel 9, jedoch mit zusätzlich zwei Amplifikationsprimern e (26 Nucleotide) und f (33 Nucleotide) sowie einer erhöhten Adapterprimer-Konzentration von 0,2 µM wurden variierende Verlängerungsprimer Konzentrationen eingestellt. Bezüglich der Amplifikationsprimer wird auf die Figur 1 verwiesen, dort BIOR und BIOF. BIOF ist ein biotinmarkierter Forwardprimer und BIOR ein biotinmarkierter Reverseprimer. Die Struktur ist in der Fig. 7 dargestellt.

Ein minimaler Bedarf an teurem Verlängerungsprimer ergab sich, wenn zunächst 25 Zyklen ohne Amplifikationsprimer und anschließend weitere 25 Zyklen mit Amplifikationsprimer gefahren wurden. Die Konzentration an Verlängerungsprimer konnte durch den Einsatz der Amplifikationsprimer bis auf 0,025 µM gesenkt werden, ein Faktor von ca. 1/20, bei dennoch verbesserter Homogenität und Ausbeute an PCR Produkt.

Diese Vorteile beruhen darauf, daß die Wahrscheinlichkeit der Bildung von Zwischenprodukten in hohen Konzentrationen mit dem Einsatz der sechs Primer stark reduziert ist, da die Primer, die zur Entstehung der Zwischenprodukte erforderlich sind, in geringen Konzentrationen eingesetzt werden. Zwischenprodukte können folglich nicht mit den Amplifikationsprimern exponentiell angereichert werden.

### Beispiel 11: PCR mit sechs Primern in zwei Stufen.

Grundsätzlich wird mit den Materialien, wie vorstehend angegeben, gearbeitet. Zunächst wird eine Vor-PCR in einem Reaktionsvolumen von 5 µl mit 0,1 pg Nukleinsäurebasissequenz durchgeführt, und zwar mit 0,3 µM Adapterprimer und 0,5 µM Verlängerungsprimer über 20 Zyklen. Dann wird die so erhaltene Reaktionslösung mit PCR Ansatzvolumen auf 25 µl verdünnt. Dann wird Amplifikationsprimer auf eine Endkonzentration von 0,5 µM zugegeben. Schließlich wird für weitere 30 Zyklen amplifiziert.

### Beispiel 12: Stabilisierung einer Nukleinsäure mit Biotin.

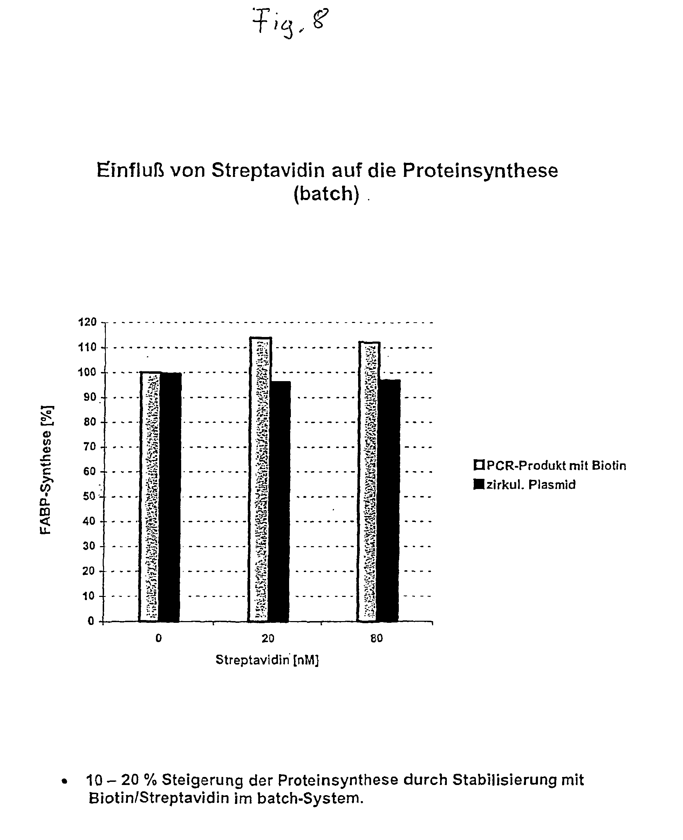

Es wurde unter Einsatz der Primer BIOF und BIOR im Wege der PCR mit 6 Primern, wie vorstehend beschrieben, eine Nukleinsäure hergestellt und deren Abbau als Funktion der Zeit sowie die Verbesserung der Proteinsynthese untersucht. Dies ist in den Figuren 7 und 8 dargestellt. Man erkennt, daß sich mit Biotin, insbesondere nach Umsatz mit Streptavidin eine erheblich verbesserte Stabilität ergibt. Dies führt auch zu einer bis zu 20% höheren Proteinsynthese, und dies in einem System mit geringen Anteilen von Exonukleasen. Das Beispiel belegt somit, daß sogar in solchen Systemen eine Verbesserung der Proteinsyntheseleistung erreicht wird. In Systemen mit Lysaten, welche höhere Mengen an Exonukleasen ausweisen, sind Verbesserungen der Syntheseleistung um einen Faktor von bis zu 5 und mehr zu erwarten.

Unabhängig von den vorstehenden Beispielen ist anzumerken, daß mit dem erfindungsgemäßen Verfahren auch sehr leicht Variationen der Sequenzen durch Mutationen möglich sind, beispielsweise durch Einsetzen von Taq-Polymerase und/oder veränderten Reaktionsbedingungen. Wenn dies nicht gewünscht ist, so kann vorzugsweise mit Pwo oder Pfu gearbeitet werden, welche genauer funktionieren und proofreading Aktivität haben.

## Patentansprüche

1. Verwendung einer gegen Abbau durch Exonukleasen stabilisierten linearen Nukleinsäure in einem Verfahren zur Herstellung eines durch eine Codesequenz der Nukleinsäure codierten Proteins in einem zellfreien Proteinbiosynthesesystem, wobei die Nukleinsäure die folgenden Komponenten aufweist:
a) eine für ein definiertes Peptid oder Protein codierende Codesequenz,
b) optional, eine die Expression der Codesequenz kontrollierende Promotorsequenz, und
c) an beiden Enden der linearen Nukleinsäure jeweils angefügte: Moleküle. A, welche jeweils mit einem nicht-immobilisierten Molekül B verbunden sind,
wobei das Molekül A ein vergleichsweise kleines Molekül ist, welches zumindest eine freie Bindungsstelle für ein zum Molekül A gepaartes Molekül B aufweist, und
wobei das Molekül B ein Protein mit einem Molekulargewicht von mehr als 1000 ist.

2. Verwendung nach Anspruch 1, wobei zwischen den Komponenten a und/oder b und den Molekülen A eine Spacersequenz eingerichtet ist.

3. Verwendung nach einem der Ansprüche 1 oder 2, **dadurch gekennzeichnet, dass** die Proteinexpression in einem zellfreien prokaryontischen System vorgenommen wird.

4. Verwendung nach Anspruch 3, **dadurch gekennzeichnet, dass** die Proteinexpression in einem zellfreien Translationssystem aus Escheria coli D10 vorgenommen wird.

5. Verwendung nach einem der Ansprüche 1 bis 4, **dadurch gekennzeichnet, dass** an beiden Enden der linearen Sequenz enthaltend die Komponenten a und b ein Biotin als Molekül A angefügt wird.

6. Verwendung nach Anspruch 5, **dadurch gekennzeichnet, dass** das Molekül B Avidin oder Streptavidin ist.

7. Verwendung nach einem der Ansprüche 1 bis 6, **dadurch gekennzeichnet, dass** an beiden Enden der linearen Sequenz enthaltend die Komponenten a und b ein Molekül Digoxygenin angefügt wird und dass das Molekül B ein Anti-Digoxygenin-Antikörper ist.

## Claims

1. The use of a linear nucleic acid, which is stabilized against decomposition by exonucleases, in a method for making a protein coded by a code sequence of the nucleic acid in a cell-free protein biosynthesis system, said nucleic acid containing the following constituents:
a) a code sequence coding for a defined peptide or protein,
b) optionally, a promoter sequence controlling the expression of the code sequence, and
c) molecules A respectively added to both ends of the linear nucleic acid, said molecules A each being linked to a non-immobilized molecule B,
wherein the molecule A is a relatively small molecule, which has at least one free binding site for a molecule B coupled to molecule A, and
wherein the molecule B is a protein having a molecular weight of more than 1,000.

2. The use according to claim 1, wherein between the constituents a and/or b and the molecules A a spacer sequence is provided.

3. The use according to one of claims 1 or 2, **characterized by** that the protein expression is carried out in a cell-free prokaryotic system.

4. The use according to claim 3, **characterized by** that the protein expression is carried out in a cell-free translation system of Escherichia coli D10.

5. The use according to one of claims 1 to 4, **characterized by** that at both ends of the linear sequence containing the constituents a and b a biotin is added as molecule A.

6. The use according to claim 5, **characterized by** that the molecule B is avidin or streptavidin.

7. The use according to one of claims 1 to 6, **characterized by** that at both ends of the linear sequence containing the constituents a and b a molecule digoxygenin is added, and that the molecule B is an anti-digoxygenin antibody.

## Revendications

1. Utilisation d'un acide nucléique linéaire, qui est stabilisé contre la décomposition par des exonucléases, dans un procédé de fabrication d'une protéine codée par une séquence de code de l'acide nucléique dans un système de biosynthèse des protéines sans cellules, cet acide nucléique contenant les composants suivants:
a) une séquence de code codant pour un peptide défini ou une protéine définie,
b) optionnellement, une séquence promotrice contrôlant l'expression de la séquence de code, et
c) des molécules A respectivement ajoutées aux deux extrémités de l'acide nucléique linéaire, ces molécules A étant liées chacune à une molécule B non immobilisée,
dans laquelle la molécule A est une molécule relativement petite, qui a au moins un site de liaison libre pour une molécule B couplée à la molécule A, et
dans laquelle la molécule B est une protéine ayant un poids moléculaire de plus de 1000.

2. Utilisation selon la revendication 1, dans laquelle entre les composants a et/ou b et les molécules A une séquence espaceuse est prévue.

3. Utilisation selon une des revendications 1 ou 2, **caractérisée en ce que** l'expression de protéine est effectuée dans un système procaryote sans cellules.

4. Utilisation selon la revendication 3, **caractérisée en ce que** l'expression de protéine est effectuée dans un système de traduction sans cellules d'Escherichia coli D10.

5. Utilisation selon une des revendications 1 à 4, **caractérisée en ce qu'**aux deux extrémités de la séquence linéaire contenant les composants a et b une biotine est ajoutée comme molécule A.

6. Utilisation selon la revendication 5, **caractérisée en ce que** la molécule B est l'avidine ou la streptavidine.

7. Utilisation selon une des revendications 1 à 6, **caractérisée en ce qu'**aux deux extrémités de la séquence linéaire contenant les composants a et b une molécule digoxygénine est ajoutée, et que la molécule B est un anticorps anti-digoxygénine.
